# EUROPEAN PATENT APPLICATION

(11) **EP 4 610 619 A1**
(43) Date of publication of application: **03.09.2025**
(21) Application number: 25160076.3
(22) Date of filing: 25.02.2025
(51) Int. Cl.: G01M 99/00, A61M 5/20, B25J 11/00, G09B 23/28, B25J 15/00

(54) **GRIPS FOR INJECTOR TESTING SYSTEMS**

(30) Priority: 28.02.2024 US 202463558971 P; 18.02.2025 US 202519056064
(71) Applicant: Illinois Tool Works Inc., Glenview IL 60025 (US)
(72) Inventor: DIEP, Sonny, Glenview, 60025 (US)
(74) Representative: HGF

(57) **Abstract**

An example injection needle positioner includes: a first grip comprising a first grip face on a first lateral side of the first grip; and a second grip, comprising: a second grip face on a first lateral side of the second grip, the second grip face facing the first grip face, the second grip face comprising: a first section that extends from a first end of the second grip and comprises a first profile configured to align an injector; and a second section located toward a second end of the first grip, relative to the first section, and having a second profile configured to securably grip the injector in cooperation with the first grip face.

## Description

### RELATED APPLICATIONS

The present application claims the benefit of U.S. Provisional Patent Application Serial No. 63/558,971, filed February 28, 2024, entitled "GRIPS FOR INJECTOR TESTING SYSTEMS." The entirety of U.S. Provisional Patent Application Serial No. 63/558,971 is expressly incorporated herein by reference.

### FIELD OF THE DISCLOSURE

This disclosure relates generally to injection device testing, and more particularly, to grips for injection testing systems.

### BACKGROUND

Injection testing systems may test one or more aspects of injection devices, including autoinjectors, for aspects such as cap removal force, plunger actuation force, injection depth, injection time, needle retraction, and/or delivered dose.

### SUMMARY

Grips for injection testing systems are disclosed, substantially as illustrated by and described in connection with at least one of the figures, as set forth more completely in the claims.

### BRIEF DESCRIPTION OF THE DRAWINGS

These and other features, aspects, and advantages of the present disclosure will become better understood when the following detailed description is read with reference to the accompanying drawings, wherein:
FIG. 1 is an example injection testing system to perform testing of injection devices, in accordance with aspects of this disclosure.
FIG. 2 is a block diagram of an example injection testing system including an injection needle positioner and grips, in accordance with aspects of this disclosure.
FIG. 3A and 3B are perspective views of an example grip that may be used to implement one of the grips of FIG. 2.
FIG. 4A is a side view of the example grip of FIG. 3A and 3B in a first orientation; FIG. 4B is a side view of the example grip of FIG. 3A and 3B in a second orientation.
FIG. 5A and 5B are perspective views of an exa7mple injection needle positioner having example grips attached to mounting blocks, in accordance with aspects of this disclosure.
FIG. 6 is a side view of the example injection needle positioner of FIG. 5A and 5B in the closed position when coupled to the mounting brackets in a first orientation.
FIG. 7 is a side view of the example injection needle positioner of FIG. 5A and 5B in the closed position when coupled to the mounting brackets in a second orientation.
FIGS. 8A and 8B illustrate another example grip which may be used to implement the grips of FIG. 2.
FIG. 9 illustrates another example injection needle positioner that may be used to implement the injection needle positioner of FIG. 2, including two grips coupled to the injection needle positioner through two mounting brackets.

The figures are not necessarily to scale. Wherever appropriate, similar or identical reference numerals are used to refer to similar or identical components.

### DETAILED DESCRIPTION

Injection needle testing devices that measure the dosage delivered by the injection needle, particularly by autoinjectors, include an injection needle positioner that is configured to align and position an injector (e.g., an autoinjector) securely and in a position suitable for various forms of testing and measurements of the injector. Conventional injection needle testing systems are unable to easily interface with certain types of injectors due to the geometry of the injector.

Disclosed example injection needle testing devices and injection testing systems include one or more grips and that provide excellent gripping characteristics. Some example injection needle testing systems include an injection needle positioner capable of aligning an injector.

Disclosed example injection needle positioner include: a first grip including a first grip face on a first lateral side of the first grip; and a second grip, including: a second grip face on a first lateral side of the second grip, the second grip face facing the first grip face, the second grip face including: a first section that extends from a first end of the second grip and includes a first profile configured to align an injector; and a second section located toward a second end of the first grip, relative to the first section, and having a second profile configured to securably grip the injector in cooperation with the first grip face.

In some example injection needle positioners, the first grip face includes a third section that extends from a first end of the first grip and having a third profile configured to align the injector, and a fourth section toward a second end of the first grip, relative to the third section, and having a fourth profile configured to securably grip the injector in cooperation with the third section and the second grip face.

In some example injection needle positioners, the first profile of the second grip includes an angled alignment portion. In some example injection needle positioners, the angled alignment portion extends toward a second lateral side of the second grip opposite the first lateral side.

In some example injection needle positioners, the second profile of the second grip includes an angled gripping portion. In some example injection needle positioners, the angled gripping portion extends at an angle away from a second lateral side of the second grip opposite the first lateral side. In some example injection needle positioners, the angle is less than 90 degrees relative to the second lateral side of the second grip. In some example injection needle positioners, the angled gripping portion extends away from and perpendicular to the second lateral side of the second grip.

In some example injection needle positioners, the first profile of the second grip is configured to align the injector having an asymmetric geometry. In some example injection needle positioners, the first grip is configured to align the injector having an asymmetric geometry. In some example injection needle positioners, the first profile of the second grip is configured to align the injector having a symmetric geometry. In some example injection needle positioners, the symmetric geometry is a cylindrical, elliptical, or semi-square geometry. In some example injection needle positioners, the first grip is configured to align the injector having a symmetric geometry. In some example injection needle positioners, the symmetric geometry is a cylindrical, elliptical, or semi-square geometry.

In some example injection needle positioners, at least a portion of at least one of the first grip face or the second grip face includes a slip-resistant coating.

In some example injection needle positioners, each grip further includes one or more accessory mounting holes. In some example injection needle positioners, the one or more accessory mounting holes are configured to interface with one or more customization plates.

In some example injection needle positioners, each grip further includes a first mounting groove configured to align with a mounting bracket. In some example injection needle positioners, each grip further includes one or more mounting holes configured to align with one or more mounting openings on the mounting bracket. In some example injection needle positioners, the first section of the second grip face includes a first alignment surface on a first side of the mounting groove and a second alignment surface on a second side of the mounting groove, in which the first and second alignment surfaces have a same profile.

Disclosed example injection needle testing system include: a positioning plate having a first side configured to contact an injector having a second side opposite the first side; an injection needle positioner configured to align the injector with at least one of a second injection needle positioner or the positioning plate, the injection needle positioner including: a first grip including a first grip face on a first lateral side of the first grip; and a second grip, including: a second grip face on a first lateral side of the second grip, the second grip face facing the first grip face, the second grip face including: a first section that extends from a first end of the second grip and having a first profile configured to align the injector; and a second section toward a second end of the first grip, relative to the first section, and having a second profile configured to securably grip the injector in cooperation with the first section and the first grip face; and an injector actuator configured to actuate the injector to expel contents of the injector via a needle of the injector.

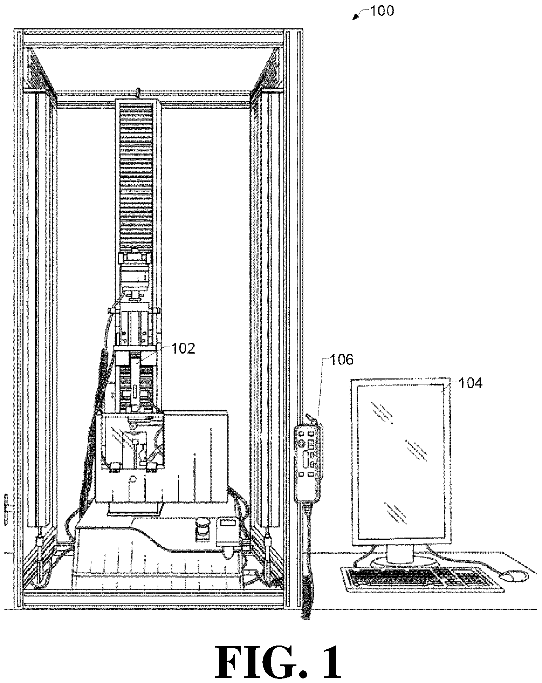

FIG. 1 is an example injection testing system 100 to perform testing on injection devices, such as an autoinjector 102. The example injection testing system 100 may be configured, for example, to perform some or all tests to evaluate requirements of the ISO 11608-5 standard. The example injection testing system 100 may be, for example, a universal testing system configured for injection testing.

The example injection testing system 100 of FIG. 1 includes positioning device(s) (e.g., to position the autoinjector 102 in one or more positions and/or orientations for automated testing), actuator(s) (e.g., to actuate components of the autoinjector 102, to actuate the positioning device(s), to position and/or orient test devices, etc.), and/or sensors to measure aspects of the autoinjector 102 during testing (e.g., load sensors to measure actuation force(s), auditory sensors to detect audible events), mass scales to measure an expelled dose, displacement and/or position sensors to trigger testing steps and/or measure displacement of components of the autoinjector 102, etc.). The example injection testing system may include one or more cameras to, for example, record injection events and/or perform optical measurements of needle extension and/or injection duration. The example injection testing system 100 further includes one or more user interface devices, such as display 104 and input devices 106.

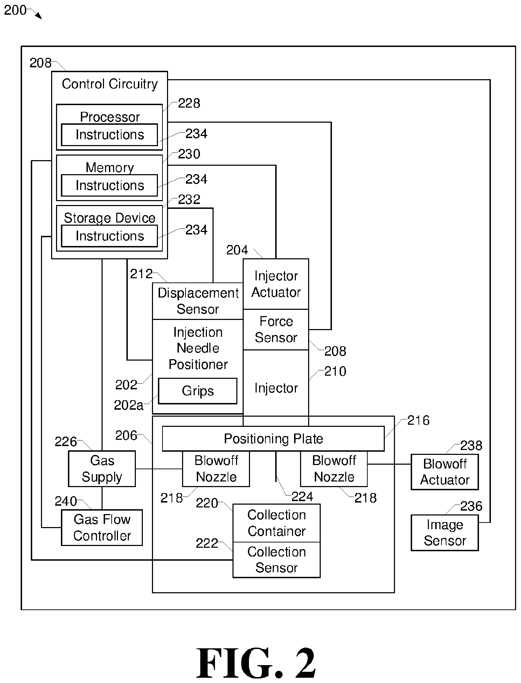

FIG. 2 is a block diagram of an example injection testing system 200 that may be used to implement some or all of the components of the injection testing system 100 of FIG. 1.

The example injection testing system 200 includes an injection needle positioner 202, an injector actuator 204, an injection collector 206, and control circuitry 208. The injection needle positioner 202 positions and/or orients an injector 210 (e.g., an autoinjector) for one or more tests in the injection testing system 200. For example, the injection needle positioner 202 may grasp the injector 210 and move and/or rotate the injector 210 for testing. The injection needle positioner may grasp the injector 210 through two or more grips 202a, which may be arranged as one or more sets of grips 202a. The injection needle positioner 202 and/or the position of the injector 210 may be measured by one or more displacement sensor(s) 212, which provides displacement and/or position information to the control circuitry 208.

The injector actuator 204 actuates one or more aspects of the injector 210, such as a plunger or other injection mechanism of the injector 210. The force applied by the injector actuator 204 may by measured by a force sensor 214, which provides force measurements to the control circuitry 208.

The injection collector 206 includes a loading surface (e.g., a positioning plate 216), blowoff nozzles 218, and a collection container 220. The collection container 220 and the injector 210 are positioned such that, when the injector 210 is actuated to expel fluid contained in the injector 210, the fluid is expelled into the collection container 220. A collection sensor 222 measures the mass and/or volume collected in the collection container 220, and provides a mass or volume measurement to the control circuitry 208.

The positioning plate 216 allows a needle 224 of the injector 210 to extend through the positioning plate 216 toward the collection container 220. The positioning plate 216 may block a body of the injector 210 from extending through the positioning plate 216 using appropriately sized apertures for the needle 224 and the body of the injector 210. To test the dispensing of the contained fluid, the injection needle positioner 202 may position the injector 210 to contact or abut the positioning plate 216, such that the needle 224 extends through the aperture of the positioning plate 216. When the injector 210 is positioned, the injector 210 may be actuated (e.g., manually, or automatically via the injector actuator 204) to expel the contents of the injector 210 into the collection container 220.

While the examples disclosed herein use the positioning plate 216 as a loading surface, other examples may use different types of loading surfaces against which the injector 210 can be actuated to expose the needle 224 and/or expel the contents of the injector 210. For example, rods or other structural members which are positioned to contact a body of the injector 210 on a top side of the loading surface, and to avoid obstructing the needle 224 may be used. In some such examples, the blowoff nozzles 218 may be coupled to another surface, or otherwise adjustably supported, within the injection collector 206 adjacent the bottom side of the loading surface and/or adjacent the location the needle 224.

At the completion of actuation of the injector 210, the blowoff nozzles 218 are controlled to blow the last drop of fluid from at or near the tip of the needle into the collection container 220. A gas supply 226 provides a gas, such as nitrogen or air, to the blowoff nozzles 218. The gas supply 226 may be, for example, a compressed gas source, a pneumatic pump, or a blower. The blowoff nozzles 218 may be positioned and/or oriented to adjust a location at which the blowoff gas strikes the needle 224, and/or may be positioned and/or oriented to cause the blowoff gas to strike the needle 224 over a range of needle lengths.

The example control circuitry 208 may be a general-purpose computer, a laptop computer, a tablet computer, and/or any other type of processing system configured to communicate with the sensors and actuators of the injection testing system 200. For example, the control circuitry 208 includes a processor 228, memory 230, and a storage device 232. The example processor 228 may be any general purpose central processing unit (CPU) from any manufacturer. In some other examples, the processor 228 may include one or more specialized processing units, such as RISC processors with an ARM core, graphic processing units, digital signal processors, and/or system-on-chips (SoC). The processor 228 executes machine readable instructions 234 that may be stored locally at the processor (e.g., in an included cache or SoC), in the memory (e.g., a random access memory or other volatile memory, a read only memory or other non-volatile memory such as FLASH memory, and/or in the storage device 232. The example storage device 232 may be a hard drive, a solid state storage drive, a hybrid drive, a RAID array, and/or any other mass data storage device.

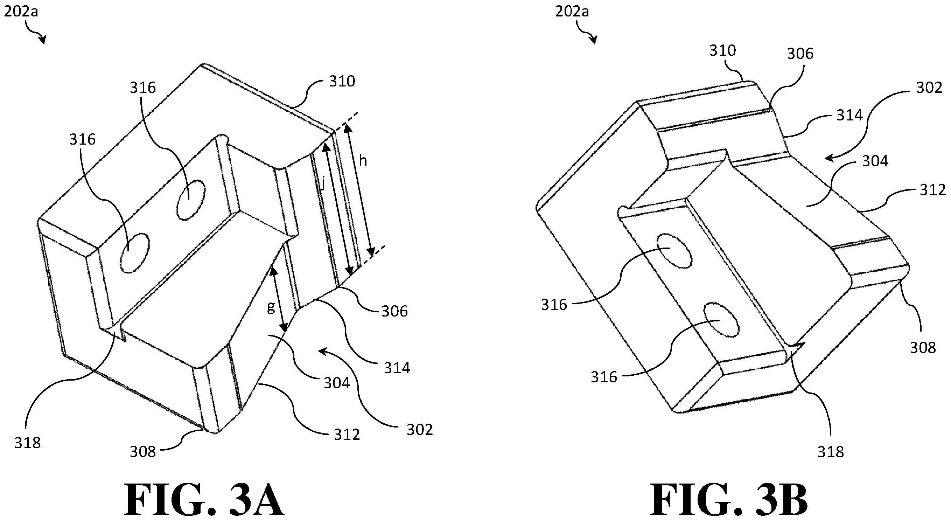

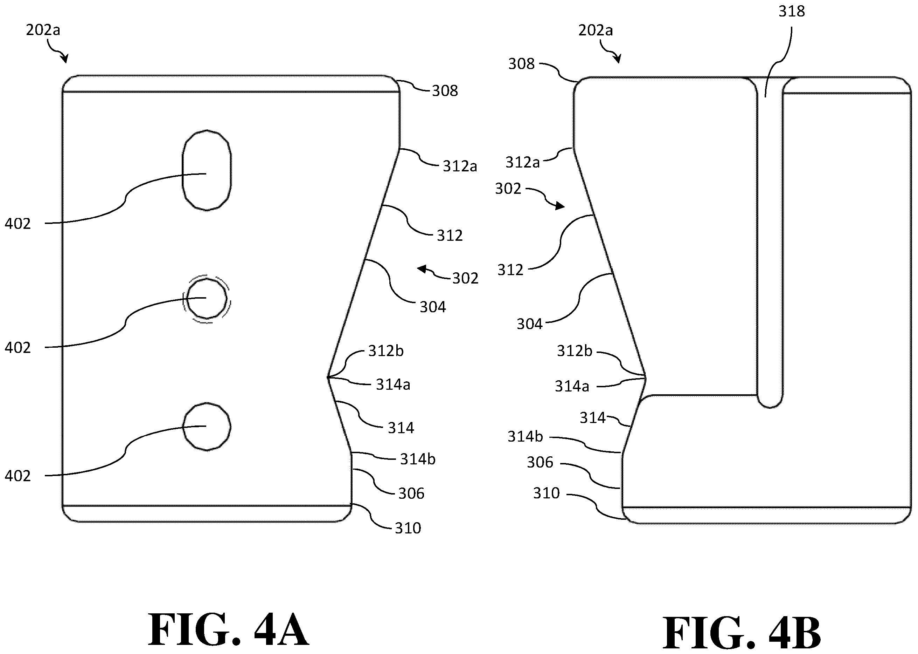

FIG. 3A and 3B are perspective views of an example grip 202a. As discussed above, injection needle positioner 202 may include one or more grips 202a through which injection needle positioner 202 may grasp an injector 210. FIG. 4A is a side view of the example grip of FIG. 3A and 3B in a first orientation, and FIG. 4B is a side view of the example grip of FIG. 3A and 3B in a second orientation.

The example grip 202a includes a grip face 302 on a first lateral side opposite a second lateral side of the grip 202a. The example grip face 302 includes two or more sections, such as a first section 304 and a second section 306. The first section 304 extends from or toward a first end 308 of the grip 202a, and the second section 306 extends from or toward a second end 310 of the grip 202a. Each section 304, 306 has a profile that is configured to interface with an injector 210. The first section 304 and the second section 306 of the example grip 202a have different profiles to interface with an injector 210. The example first section 304 has a partial height g that is less than a height h of the grip face 302 (e.g., g < h), and the second section 306 has height j that extends the full height h of the grip face 302 (e.g., j = h)

As described above, the profile of each section 304, 306 of the example grip 202a is configured to interface with an injector 210. To that end, the example first section 304 includes a profile that is configured to align an injector 210 as the grip 202a engages with the injector 210. For example, as the grips 202a converge on and physically engage the injector 210 (e.g., with a first and second grip 202a, wherein the first and second grips are facing each other) the first section 304 urges the injector 210 to align into a desirable position (e.g., aligned with a gripping portion of the grip 202a, such as an angled gripping portion 314) by applying force to the surface of the injector 210 to adjust the orientation of the injector 210 until the surface of the injector 210 contacts the first section 304 substantially evenly. In some examples, the alignment portion of first section 304 is an angled alignment portion 312 for at least one of the opposing grips 202a. The angled alignment portion 312 of FIGS. 3A and 3B extends at an angle toward the second lateral side that is opposite the first lateral side of grip 202a (e.g., from a first end 312a to a second end 312b of the angled alignment portion 312). While an example angle of the angled alignment portion 312 is illustrated in FIGS. 3A, 3B, 4A, 4B, 5A, 5B, 6, and 7, different angles may be used. In other examples, the alignment portion 312 may comprise other profiles, such as a curved alignment portion.

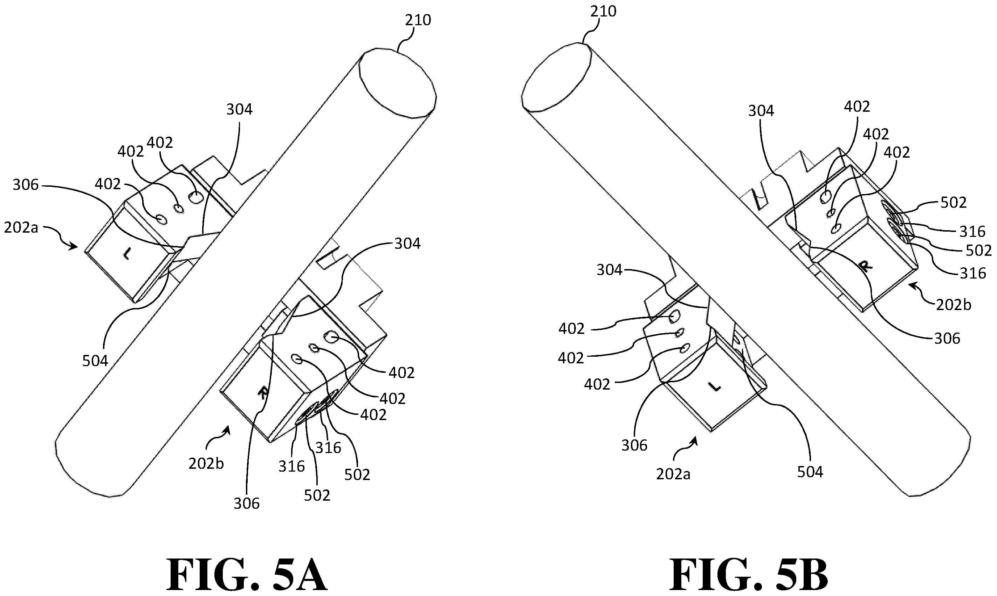

The second section 306 includes a second profile that is configured to securably grip an injector 210. To that end, example second profiles of the second section 306 are configured to securably grip an injector 210 in cooperation with another grip face 302 (e.g., a first grip 202a acting in cooperation with a second grip 202a, wherein the first and second grips are facing each other). The example second profile includes an angled gripping portion 314. Thus, example grips 202a can be configured to securably grip an injector 210 using an angled gripping portion 314 of the grips 202a, as illustrated in FIG. 5A, 5B, 6, and 7. In some examples, angled gripping portion 314 extends at an angle away from the second lateral side that is opposite the first lateral side of grip 202a (e.g., from a first end 314a to a second end 314b of the angled gripping portion 314). While an example angle of the angled gripping portion 314 is illustrated in FIGS. 3A, 3B, 4A, 4B, 5A, 5B, 6, and 7, different angles may be used. For example, the angled gripping portion 314 can extend away from the second lateral side of the grip 202a at an angle of less than 90 degrees relative to the second lateral side of the grip 202a (e.g., 80, 70, 60, 50, 40, 30, 20, or 10 degrees). In other examples, the angled gripping portion 314 extends away from and perpendicular to the second lateral side of the grip 202a. Thus, the relative angles of the angled alignment portion 312 and angled gripping portion 314 can be oriented to form a v-shaped surface configured to securably grip an injector 210. In further examples, the gripping portion may comprise other profiles, such as a curved gripping portion.

As described above, the profile of each section 304, 306 of the example grip 202a is configured to interface with an injector 210. To that end, the shape of the profile may be varied depending on the shape of the injector 210. For example, each section 304 and 306 may be configured to interface with an injector 210 that has an asymmetric geometry. Thus, example grips 202a are configured to align (e.g., with angled alignment portion 312) and grip (e.g., with the angled alignment portion 312 and the angled gripping portion 314) an injector 210 that has an asymmetric geometry. In further examples, each section 304 and 306 may be configured to interface with an injector 210 that has a symmetric geometry. Thus, example grips 202a are configured to align (e.g., with angled alignment portion 312) and grip (e.g., with angled gripping portion 314) an injector 210 that has a symmetric geometry. Example symmetric geometries of the injector 210 include cylindrical, elliptical, and semi-square geometries.

In some examples, the grip 202a is be made from a material that is uncoated. In other examples, at least a portion of grip 202a includes a slip-resistant coating. The slip-resistant coating of example grip 202a is configured to enhance the stability of injector 210 when engaged by two grips 202a. Various slip-resistant coatings may be included. In some examples the slip-resistant coating is a surfalloy coating, rubber, brake lining materials, and/or any other appropriate coating material. Additionally or alternatively, the grip face 302 may be knurled, or otherwise textured to increase friction between the grip 202a and the injector 210 without coating. The grip 202a, including the grip face 302, may also be bead blasted, passivated, and/or otherwise treated to improve aesthetics and/or corrosion resistance.

The grip 202a may include one or more mounting grooves 318 through which the grip 202a can interface with or be coupled to the injection needle positioner 202. For example, the grip 202a may include a single mounting groove 318. The mounting groove 318 is configured to interface with, or be coupled to, a mounting bracket 504 of the injection needle positioner 202 as described below. The mounting groove 318 may extend through a portion of the length of the grip 202a or throughout the entire length of the grip 202a. The size and shape of the mounting groove 318 is selected to accommodate the shape and orientation of the mounting brackets 504. For example the mounting groove 318 may be square, semi-square, rectangular, circular, elliptical, or any other suitable shape.

The grip 202a may include one or more accessory mounting holes 402. The accessory mounting holes 402 may be threaded and configured to interface with a threaded mounting screw. The accessory mounting holes 402 may be configured to interface with or couple to various accessories to either side of example grip 202a. For example, the accessory mounting holes 402 may be configured to interface with one or more customization plates. In some examples, the one or more customization plates are configured to alter, customize, and/or replace the profile of the first and/or second sections 304 and 306 of the grip face 302. For example, a custom plate may be attached to each of the grips 202a via the accessory mounting holes 402. The profile of the custom plate may be configured to engage the injector 210 prior to engagement of the injector 210 by the grips 202a, which causes the custom plate to perform the gripping of the injector 210. The custom plates may be coated for improved grip as disclosed above. As described above, the shape of the profile may be configured to interface with an injector 210 having a certain geometry, such as a symmetrical or asymmetrical geometry. The grips 202a may include more or fewer accessory mounting holes 402 than shown in the illustrated examples. For instance, the grips 202a may include one or more additional alignment holes, which may be engaged by the custom plates and/or by an alignment object (e.g., a pin) which aids in appropriate alignment of the custom plates.

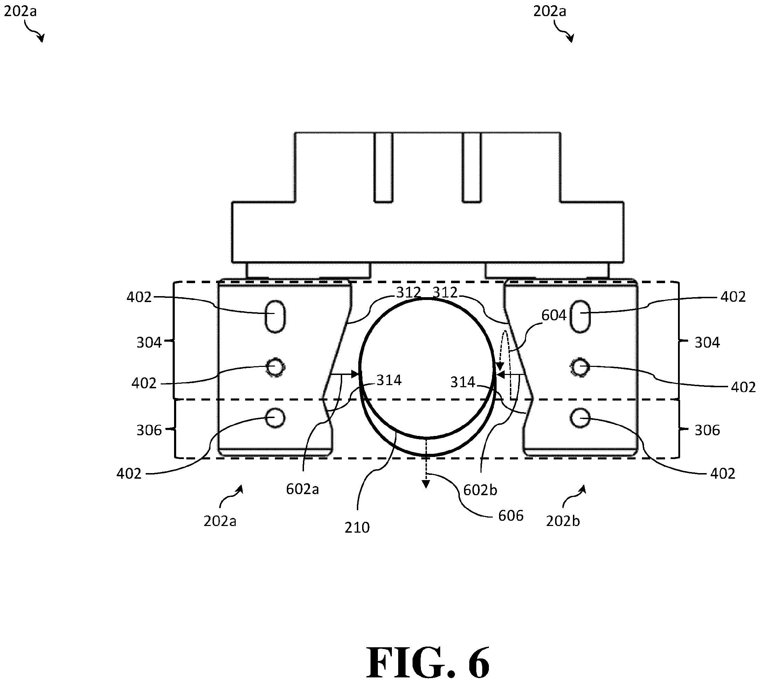

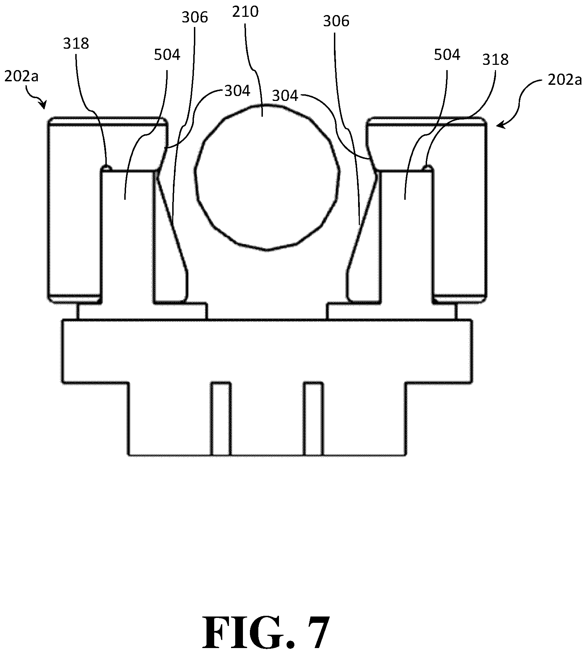

FIG. 5A and 5B are perspective views of an example injection needle positioner 202, including grips 202a coupled to the injection needle positioner 202 through mounting brackets 504. FIG. 6 is a side view of the example injection needle positioner of FIG. 5 where the grips 202a are in a partially closed position when coupled to the mounting brackets in a first orientation. FIG. 7 is a side view of the example injection needle positioner of FIG. 5 where the grips 202a are in a partially closed position when coupled to the mounting brackets in a second orientation. As shown in FIG. 5A and 5B, the example grip 202a includes one or more mounting holes 316 configured to interface with or couple to a mounting bracket 504. The mounting holes may be oriented perpendicular to the mounting groove 318. In some examples, the mounting holes 316 are threaded and configured to interface with a threaded mounting screw 502 to couple to mounting bracket 504. In further examples, one or more threaded mounting screws 502 may interface with one or more mounting openings located on the mounting bracket 504 to securely couple the grip 202a to the mounting bracket 504.

As shown in FIG. 5A and 5B, the example injection needle positioner 202 includes two grips 202a, 202b coupled to the injection needle positioner 202 through two mounting brackets 504. The example grips 202a, 202b have mirrored arrangements. When in the open position, there is space between the grips 202a, 202b such that the grips 202a, 202b are oriented to receive an injector 210 (e.g., an autoinjector). The two grips 202a, 202b may be moved laterally towards one into a closed position. When in the closed position, the example grips 202a, 202b are configured to securely grip an injector 210. As illustrated in FIG. 5A, 5B, 6, and 7, each grip 202a of the injection needle positioner 202 may include some or all of the components of the grip 202a as described above and illustrated in FIG. 1-4.

FIG. 6 illustrates a misaligned injector 210 in position between the example grips 202a, 202b. As the grips 202a, 202b close toward the injector 210 (as indicated by arrows 602), the alignment portions 312 of the respective grips 202a, 202b align the injector 210 (as shown by the direction of rotation 604 or, alternatively, by urging the upper portion of the injector 210 in the direction 606). As the grips 202a, 202b fully close on the injector 210, the injector 210 is held in the desired position by the alignment portions 312 and the gripping portions 314 of the grips 202a, 202b.

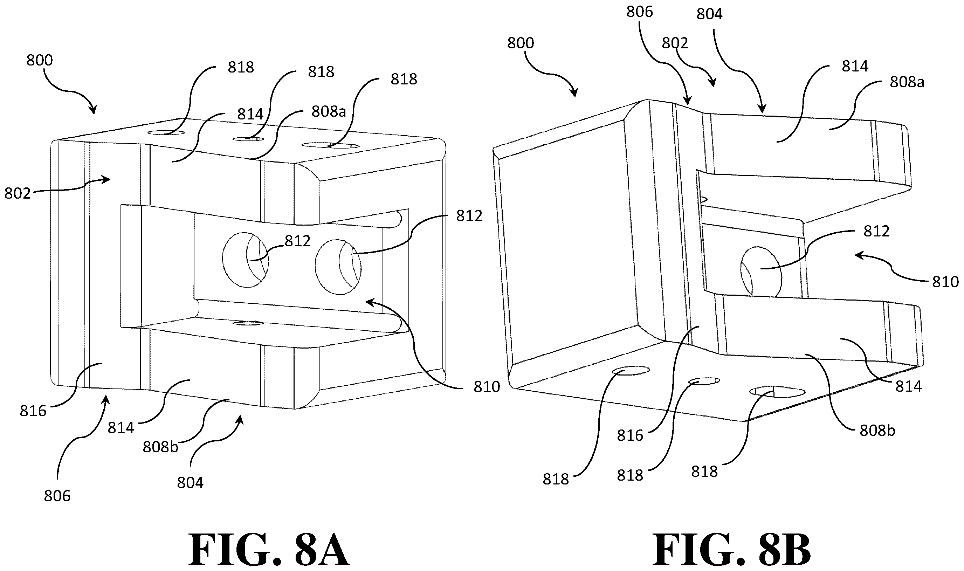

FIGS. 8A and 8B illustrate another example grip 800 which may be used to implement the grips 202a, 202b of FIG. 2. The example grip 800 includes a grip face 802 having a first section 804 and a second section 806. The grip face 802, the first section 804, and the second section 806 provide the same alignment and gripping functions as the grip face 302, the first section 304, and the second section 306 of FIGS. 3A and 3B, respectively. The grip face 802, the first section 804, and the second section 806 may include the same materials and/or coatings as disclosed above with reference to the grip face 302, the first section 304, and the second section 306. Specifically, the profile of each section 804, 806 of the example grip 800 is configured to interface with an injector 210.

In contrast with the example grip 202a of FIGS. 3A and 3B, the example first section 804 includes upper and lower surfaces 808a, 808b which are spaced apart by a mounting groove 810. The mounting groove 810 accommodates the mounting bracket 504 for attachment of the grip 800 to the injection needle position 202 via fasteners, such as mounting screws 502, and mounting holes 812. The upper and lower surfaces 808a, 808b are configured to align the injector 210 as the grip 800 engages the injector 210, in a same or similar manner as disclosed above with reference to the first section 304 of FIGS. 3A and 3B, such as by including angled alignment portions 814 and an angled gripping portion 816. The angled alignment portions 814 have a same profile as the angled alignment portions 314 of FIGS. 3A and 3B, and the angled gripping portion 816 has the same profile as the angled gripping portion 316. However, the alignment portions 814 may have other profiles.

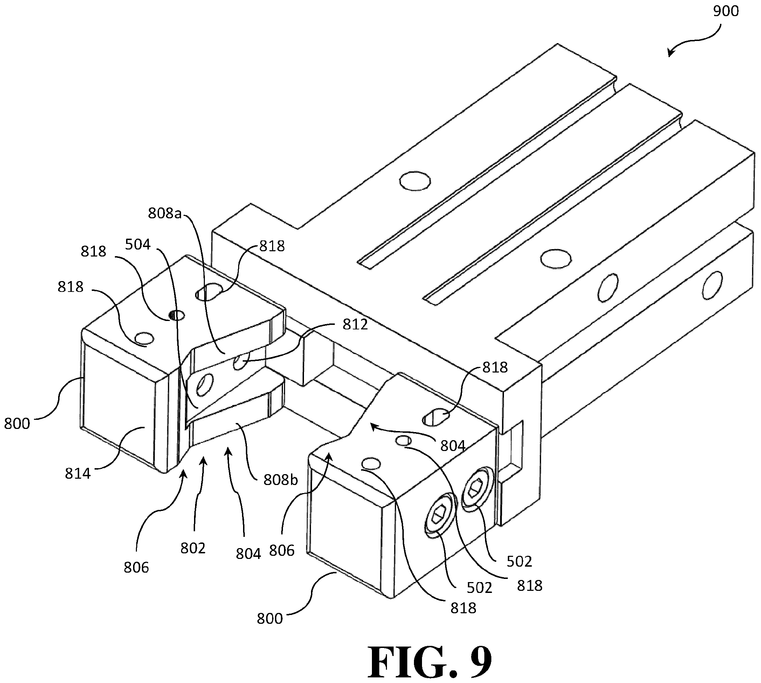

The example grip 800 is reversible, in that the grip 800 may be used on either side of the injector 210, which allows a reduction in the number of types of grips that are needed by the operator. FIG. 9 illustrates another example injection needle positioner 900 that may be used to implement the injection needle positioner 202, including two grips 800 coupled to the injection needle positioner 900 through two mounting brackets 504.

The grip 800 may include the one or more accessory mounting holes 818. The accessory mounting holes 818 may be threaded and configured to interface with a threaded mounting screw. The accessory mounting holes 818 may be configured to interface with or couple to various accessories, and may be positioned on either or both sides of the example grip 800. For example, the accessory mounting holes 818 may be configured to interface with one or more customization plates. In some examples, the one or more customization plates are configured to alter, customize, and/or replace the profile of the first and/or second sections 804 and 806 of the grip face 802. For example, a custom plate may be attached to each of the grips 800 via the accessory mounting holes 818.

The present methods and systems may be realized in hardware, software, and/or a combination of hardware and software. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise an application specific integrated circuit or chip. Some implementations may comprise a non-transitory machine-readable (e.g., computer readable) medium (e.g., FLASH drive, optical disk, magnetic storage disk, or the like) having stored thereon one or more lines of code executable by a machine, thereby causing the machine to perform processes as described herein. As used herein, the term "non-transitory machine-readable medium" is defined to include all types of machine readable storage media and to exclude propagating signals.

As utilized herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present method and/or system has been described with reference to certain implementations, it will be understood by those skilled in the art that various changes may be made and equivalents may be substituted without departing from the scope of the present method and/or system. For example, block and/or components of disclosed examples may be combined, divided, re-arranged, and/or otherwise modified. In addition, many modifications may be made to adapt a particular situation or material to the teachings of the present disclosure without departing from its scope. Therefore, the present method and/or system are not limited to the particular implementations disclosed. Instead, the present method and/or system will include all implementations falling within the scope of the appended claims, both literally and under the doctrine of equivalents.
Certain embodiments of the invention are described in the following clauses:
Clause 1. An injection needle positioner, comprising:
   a first grip comprising a first grip face on a first lateral side of the first grip; and
   a second grip, comprising:
      a second grip face on a first lateral side of the second grip, the second grip face facing the first grip face, the second grip face comprising:
      a first section that extends from a first end of the second grip and comprises a first profile configured to align an injector; and
      a second section located toward a second end of the first grip, relative to the first section, and having a second profile configured to securably grip the injector in cooperation with the first grip face.
Clause 2. The injection needle positioner of clause 1, wherein the first grip face comprises a third section that extends from a first end of the first grip and having a third profile configured to align the injector, and a fourth section toward a second end of the first grip, relative to the third section, and having a fourth profile configured to securably grip the injector in cooperation with the third section and the second grip face.
Clause 3. The injection needle positioner of clause 1, wherein the first profile of the second grip comprises an angled alignment portion.
Clause 4. The injection needle positioner of clause 3, wherein the angled alignment portion extends toward a second lateral side of the second grip opposite the first lateral side.
Clause 5. The injection needle positioner of clause 1, wherein the second profile of the second grip comprises an angled gripping portion.
Clause 6. The injection needle positioner of clause 5, wherein the angled gripping portion extends at an angle away from a second lateral side of the second grip opposite the first lateral side.
Clause 7. The injection needle positioner of clause 6, wherein the angle is less than 90 degrees relative to the second lateral side of the second grip.
Clause 8. The injection needle positioner of clause 6, wherein the angled gripping portion extends away from and perpendicular to the second lateral side of the second grip.
Clause 9. The injection needle positioner of clause 1, wherein the first profile of the second grip is configured to align the injector having an asymmetric geometry.
Clause 10. The injection needle positioner of clause 1, wherein the first grip is configured to align the injector having an asymmetric geometry.
Clause 11. The injection needle positioner of clause 1, wherein the first profile of the second grip is configured to align the injector having a symmetric geometry.
Clause 12. The injection needle positioner of clause 11, wherein the symmetric geometry is a cylindrical, elliptical, or semi-square geometry.
Clause 13. The injection needle positioner of clause 1, wherein the first grip is configured to align the injector having a symmetric geometry.
Clause 14. The injection needle positioner of clause 13, wherein the symmetric geometry is a cylindrical, elliptical, or semi-square geometry.
Clause 15. The injection needle positioner of clause 1, wherein at least a portion of at least one of the first grip face or the second grip face comprises a slip-resistant coating.
Clause 16. The injection needle positioner of clause 1, wherein each grip further comprises one or more accessory mounting holes configured to interface with one or more customization plates.
Clause 17. The injection needle positioner of clause 1, wherein the second grip further comprises a mounting groove configured to align with a mounting bracket.
Clause 18. The injection needle positioner of clause 17, wherein the second grip further comprises one or more mounting holes configured to align with one or more mounting openings on the mounting bracket.
Clause 19. The injection needle positioner of clause 17, wherein the first section of the second grip face comprises a first alignment surface on a first side of the mounting groove and a second alignment surface on a second side of the mounting groove, the first and second alignment surfaces having a same profile.
Clause 20. An injection needle testing system, comprising:
   a positioning plate having a first side configured to contact an injector having a second side opposite the first side;
   an injection needle positioner configured to align the injector with at least one of a second injection needle positioner or the positioning plate, the injection needle positioner comprising:
      a first grip comprising a first grip face on a first lateral side of the first grip; and
      a second grip, comprising:
         a second grip face on a first lateral side of the second grip, the second grip face facing the first grip face, the second grip face comprising:
         a first section that extends from a first end of the second grip and having a first profile configured to align the injector; and
         a second section toward a second end of the first grip, relative to the first section, and having a second profile configured to securably grip the injector in cooperation with the first section and the first grip face; and
   an injector actuator configured to actuate the injector to expel contents of the injector via a needle of the injector.

## Claims

1. An injection needle positioner, comprising:
a first grip comprising a first grip face on a first lateral side of the first grip; and
a second grip, comprising:
a second grip face on a first lateral side of the second grip, the second grip face facing the first grip face, the second grip face comprising:
a first section that extends from a first end of the second grip and comprises a first profile configured to align an injector; and
a second section located toward a second end of the first grip, relative to the first section, and having a second profile configured to securably grip the injector in cooperation with the first grip face.

2. The injection needle positioner of claim 1, wherein the first grip face comprises a third section that extends from a first end of the first grip and having a third profile configured to align the injector, and a fourth section toward a second end of the first grip, relative to the third section, and having a fourth profile configured to securably grip the injector in cooperation with the third section and the second grip face.

3. The injection needle positioner of claim 1 or claim 2, wherein the first profile of the second grip comprises an angled alignment portion, and optionally
wherein the angled alignment portion extends toward a second lateral side of the second grip opposite the first lateral side.

4. The injection needle positioner of any one of claims 1 to 3, wherein the second profile of the second grip comprises an angled gripping portion.

5. The injection needle positioner of claim 4, wherein the angled gripping portion extends at an angle away from a second lateral side of the second grip opposite the first lateral side.

6. The injection needle positioner of claim 5, wherein the angle is less than 90 degrees relative to the second lateral side of the second grip, or
wherein the angled gripping portion extends away from and perpendicular to the second lateral side of the second grip.

7. The injection needle positioner of any one of claims 1 to 6, wherein the first profile of the second grip is configured to align the injector having an asymmetric geometry.

8. The injection needle positioner of any one of claims 1 to 7, wherein the first grip is configured to align the injector having an asymmetric geometry.

9. The injection needle positioner of any one of claims 1 to 8, wherein the first profile of the second grip is configured to align the injector having a symmetric geometry, and optionally
wherein the symmetric geometry is a cylindrical, elliptical, or semi-square geometry.

10. The injection needle positioner of any one of claims 1 to 9, wherein the first grip is configured to align the injector having a symmetric geometry, and optionally
wherein the symmetric geometry is a cylindrical, elliptical, or semi-square geometry.

11. The injection needle positioner of any one of claims 1 to 10, wherein at least a portion of at least one of the first grip face or the second grip face comprises a slip-resistant coating.

12. The injection needle positioner of any one of claims 1 to 11, wherein each grip further comprises one or more accessory mounting holes configured to interface with one or more customization plates.

13. The injection needle positioner of any one of claims 1 to 12, wherein the second grip further comprises a mounting groove configured to align with a mounting bracket.

14. The injection needle positioner of claim 13, wherein the second grip further comprises one or more mounting holes configured to align with one or more mounting openings on the mounting bracket, or
wherein the first section of the second grip face comprises a first alignment surface on a first side of the mounting groove and a second alignment surface on a second side of the mounting groove, the first and second alignment surfaces having a same profile.

15. An injection needle testing system, comprising:
a positioning plate having a first side configured to contact an injector having a second side opposite the first side;
an injection needle positioner configured to align the injector with at least one of a second injection needle positioner or the positioning plate, the injection needle positioner comprising:
a first grip comprising a first grip face on a first lateral side of the first grip; and
a second grip, comprising:
a second grip face on a first lateral side of the second grip, the second grip face facing the first grip face, the second grip face comprising:
a first section that extends from a first end of the second grip and having a first profile configured to align the injector; and
a second section toward a second end of the first grip, relative to the first section, and having a second profile configured to securably grip the injector in cooperation with the first section and the first grip face; and
an injector actuator configured to actuate the injector to expel contents of the injector via a needle of the injector.
